# EUROPÄISCHE PATENTANMELDUNG

(11) **EP 0 700 928 A1**
(43) Veröffentlichungstag der Anmeldung: **13.03.1996**
(21) Anmeldenummer: 95111735.7
(22) Anmeldetag: 26.07.1995
(51) Int. Cl.: C07K 14/00, A61K 38/02, C12Q 1/68

(54) **Nukleinsäuren-bindende Oligomere für Therapie und Diagnostik**

(30) Priorität: 08.08.1994 DE 4427980
(71) Anmelder: BAYER AG, D-51368 Leverkusen (DE)
(72) Erfinder: Schwemler, Christoph, Dr., D-42799 Leichlingen (DE); Pötter, Thorsten, Dr., D-51061 Köln (DE); Mielke, Burkhard, Dr., D-51375 Leverkusen (DE); Schwenner, Eckhard, Dr., D-42113 Wuppertal (DE); Kretschmer, Axel, Dr., D-51429 Bergisch Gladbach (DE); Stropp, Udo, Dr., D-42781 Haan (DE); Kosch, Winfried, Dr., D-51065 Köln (DE); Dürr, Hansjörg, Dr., D-51399 Burscheid (DE)

(57) **Zusammenfassung**

Die vorliegende Erfindung betrifft Verbindungen der allgemeinen Formel (I), 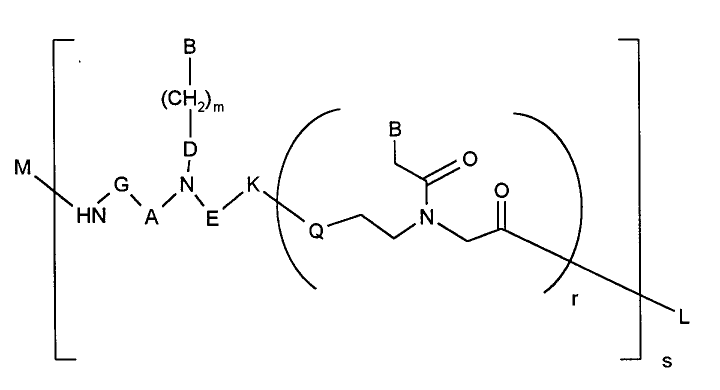
in der die Reste die in der Beschreibung angegebene Bedeutung haben, Verfahren zu ihrer Herstellung und ihre Verwendung als Arzneimittel.

## Beschreibung

Das gezielte Abschalten von Genexpression durch komplementäre Nukleinsäuren, sogenannte Antisense-Oligonukleotide, stellt einen neuen Therapieansatz dar. Mögliche Anwendungen reichen von der Behandlung viraler Infektionen bis zur Therapie von Krebs (S. Agrawal, Tibtech 10, 152 (1992); W. James, Antiviral Chemistry & Chemotherapie 2, 191(1991); B. Calabretta, Cancer Research 51, 4504 (1991)). Die Kontrolle der Genexpression erfolgt auf der Ebene von DNA und RNA und gelingt bereits mit unmodifizierten Oligonukleotiden (C. Helene, Anti-Cancer Drug Design 6, 569 (1991); E. Uhlmann, A. Peymann, Chemical Reviews 90, 543 (1990)). Diese sind jedoch aufgrund mangelnder enzymatischer Stabilität und zu geringer Aufnahme in zelluläre Systeme für therapeutische Anwendungen nicht geeignet. Therapeutische Anwendungen erfordern chemisch modifizierte Antisense-Oligonukleotide.

Neben der Antisense-Strategie kann eine Inhibition der Genexpression auch durch die Sense-Strategie erzielt werden. Dabei konkurrieren die Sense-Oligonukleotide spezifisch mit DNA-Bindungsproteinen wie z.B. Transkriptionsfaktoren (M. Blumengeld, Nucleic Acids Research 21, 3405 (1993)).

Oligonukleotide mit modifiziertem Internukleotidphosphat oder einer phosphatfreien Internukleotidverknüpfung wurden in vielen Arbeiten systematisch untersucht; ihre Synthese erwies sich jedoch als sehr aufwendig und beobachtete therapeutische Effekte als nicht ausreichend (E. Uhlmann, A. Peyman, Chemical Reviews 90, 543 (1990)).

Eine Alternative zur Modifikation oder Substitution der Phosphatgruppe in Nukleinsäuren ist der komplette Austausch von Ribose und Phosphat durch andere Rückgrate. Dieses Konzept wurde erstmals von Pitha et al. realisiert, der Ribosephosphat durch Poly-N-Vinyl-derivate ersetzte, was zu sogenannter "Plastik-DNA" führt (J. Pitha, P.O.P. Ts'O, J. Org. Chem. 33, 1341 (1968); J. Pitha, J. Adv. Polym. Sci. 50, 1 (1983)). Es erlaubt jedoch nicht den gezielten Aufbau definierter Sequenzen.

Die Synthese definierter Sequenzen gelingt, wenn anstelle von Zuckerphosphat beispielsweise ein Polyamid-Rückgrat verwendet wird, das in Analogie zur konventionellen Peptidsynthese (M. Bodanszky, Principles of Peptide Synthesis, Springer, Berlin 1984) schrittweise aufgebaut wird. Dieses Konzept wurde von verschiedenen Arbeitsgruppen unterschiedlich realisiert (J.E. Summerton et al. WO 86/05518; R. S. Varma et al. WO 92/18518; O. Buchardt et al. WO 92/20702; H. Wang, D. D. Weller, Teträhedron Letters 32, 7385 (1991); P. Garner, J. U. Yoo, Tetrahedron Letters 34; 1275 (1993); S.-B. Huang, J. S. Nelson D. D. Weller; J. Org. Chem. 56; 6007 (1991)).

Polyamid-Nukleinsäuren eignen sich ebenfalls für diagnostische und molekularbiologische Anwendungen (Buchardt et al. WO 92/20703 und Glaxo WO 93/12129).

Bei der Bearbeitung deartiger Strukturen gelang die Synthese neuer N-verzweigter oligomerer Nukleinsäuren. Für diese wurde eine überraschend gute Bindung an DNA und RNA gefunden. Die Substanzen eignen sich zur Kontrolle der Genexpression und zeigen antivirale Eigenschaften. Weiterhin lassen sich derartige Substanzen in Diagnostik und Molekularbiologie zur Isolierung, Identifizierung und Quantifizierung von Nukleinsäuren verwenden.

Die Erfindung betrifft Verbindungen der allgemeinen Formel (I), 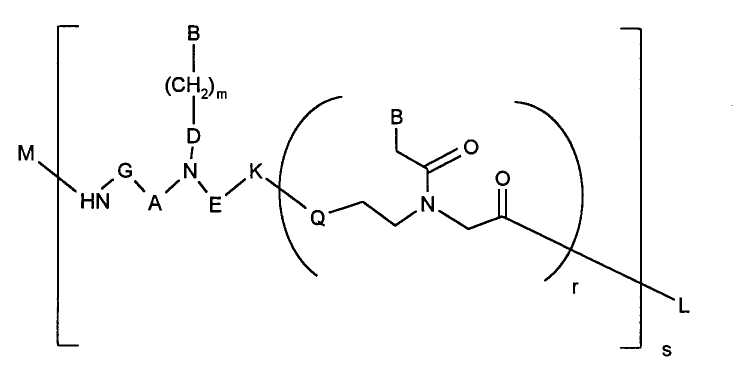
in der
- A: für -(CH₂)ₙ- oder -CO- steht,
- B: für alle natürlichen oder unnatürlichen Nukleobasen, wie z.B. Thymin, Uracil, Cytosin, Adenin, Guanin, Hypoxanthin, oder durch chemische Modifikation von diesen abgeleitete Derivate oder halogenierte Vorstufen von diesen, gegebenenfalls an den Aminogruppen mit Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert oder mit freien Aminogruppen steht,
- D: -(CO)ₚ- steht,
E und G unabhängig voneinander für -CHR- stehen, wobei
- R: Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Tyrosin, Histidin, Tryptophan, Lysin, Ornithin, Asparagin, Asparaginsäure, Glutamin, Glutaminsäure, Arginin, Prolin, Hydroxyprolin, Sarcosin, Dehydroaminosäuren, wie z.B. Dehydroalanin, Dehydro-α-aminobuttersäure, anderen unnatürlichen Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Iodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, in ihrer D- oder L-Form oder gegebenenenfalls
E und G über eine Kette -(CHR')_{q}- miteinander verknüpft sind,
- K: für -CO-, -SO₂- oder -CH₂- steht,
- L: ein Carrier-System, Reporter-Ligand, eine löslichkeitsvermittelnde Gruppe oder OH sein kann,
- M: unabhängig von L ein Carrier-System, Reporter-Ligand, eine löslichkeitsvermittelnde Gruppe oder Wasserstoff sein kann,
- Q: für NH, O, S, NR'' steht,
- R': unabhängig voneinander aus einer Gruppe bestehend aus H, OH, SH, NH₂, NHR'', N₃, Alkyl (wobei Alkyl = Methyl, Ethyl, n-Propyl, n-Butyl, iso-Butyl, tert.-Butyl oder längerkettigen verzweigten oder unverzweigten, gesättigten oder ungesättigten Alkylketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) ausgewählt werden kann,
- R'': für Schutzgruppen wie z.B. Boc, Fmoc, Z, Pyoc, Alloc oder andere in der Peptidchemie üblichen Schutzgruppen oder aber für Alkylsubstitution (wobei Alkyl = Methyl, Ethyl, n-Propyl, n-Butyl, iso-Butyl, tert.-Butyl oder längerkettigen verzweigten oder unverzweigten, gesättigten oder ungesättigten Alyklketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) steht,
- m: 0, 1, 2 oder 3 sein kann,
- n: 0, 1, 2, 3 oder 4 sein kann,
- p: 0, 1 oder 2 sein kann,
- q: 0, 1 oder 2 sein kann, und
- r: 0, oder 1 sein kann, und
- s: Werte zwischen 1 und 30 annehmen kann.

Für den Fall, daß in allen Monomeren r = 1 ist, kommt dieser Baustein alternierend vor und hat einen Anteil von 50 % am Gesamtmolekül. Wenn r in einzelnen Monomeren 0 (null) ist, reduziert sich der Anteil dieses Bausteins entsprechend auf beispielsweise 40, 30 oder 20 %. Im Gesamtmolekül soll dieser Baustein mindestens einmal vorkommen.

Bevorzugt sind Verbindungen der allgemeinen Formel (I),
in der
- A: für -(CH₂)ₙ- oder -CO- steht,
- B: für alle natürlichen Nukleobasen, wie z.B. Thymin, Uracil, Cytosin, Adenin, Guanin oder Hypoxanthin oder halogenierte Vorstufen von diesen, gegebenenfalls an den Aminogruppen substituiert durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)-methoxycarbonyl oder andere in der Peptid- und Nukleinsäurechemie übliche Schutzgruppen oder mit freier Aminogruppe steht,
- D: für -(CO)ₚ- steht,
E und G unabhängig voneinander für -CHR- stehen, wobei
- R: Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Tyrosin, Histidin, Tryptophan, Lysin, Ornithin, Asparagin, Asparaginsäure, Glutamin, Glutaminsäure, Arginin, Prolin, Hydroxyprolin, Sarcosin, Dehydroaminosäuren, wie z.B. Dehydroalanin, Dehydro-α-aminobuttersäure, anderen unnatürlichen Aminosäuren wie Phenylglycin, 2-Pyridylalanin, 3-Pyridylalanin, 4-Pyridylalanin, 1-Naphthyalanin oder 2-Naphthylalanin, gegebenenfalls mit Schutzgruppen, in ihrer D- oder L-Form oder gegebenenenfalls
E und G über eine Kette -(CHR')_{q}- miteinander verknüpft sind,
- K: -CO-, -SO₂- oder -CH₂- sein kann,
- L: ein Carrier-System, Reporter-Ligand, eine löslichkeitsvermittelnde Gruppe oder Wasserstoff sein kann,
- M: unabhängig von L ein Carrier-System, Reporter-Ligand, eine löslichkeitsvermittelnde Gruppe oder Wasserstoff sein kann,
- Q: für NH, O, NR'' steht,
- R': unabhängig voneinander aus einer Gruppe bestehend aus H, OH, SH, NH₂, NHR'', N₃, Alkyl (wobei Alkyl = Methyl, Ethyl, n-Propyl, n-Butyl, iso-Butyl, tert.-Butyl oder längerkettigen verzweigten oder unverzweigten, gesättigten oder ungesättigten Alkylketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) ausgewählt werden kann,
- R'': für Schutzgruppen wie z.B. Boc, Fmoc, Z, Pyoc, Alloc oder andere in der Peptidchemie üblichen Schutzgruppen oder aber für Alkylsubstitution (wobei Alkyl = Methyl, Ethyl, n-Propyl, n-Butyl, iso-Butyl, tert.-Butyl oder längerkettigen verzweigten oder unverzweigten, gesättigten oder ungesättigten Alyklketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) steht,
- m: 0, 1, 2 oder 3 sein kann,
- n: 0, 1, 2 oder 3 sein kann,
- p: 0 oder 1 sein kann,
- q: 0, 1 oder 2 sein kann,
- r: 0 oder 1 sein kann, und
- s: Werte zwischen 3 und 20 annehmen kann.

Mit Carrier-System oder Reporter-Ligand ist gemeint ein zellspezifisches Bindungs-und Erkennungs-Agens, das spezifisch an die Zelloberfläche bindet und das die Internalisierung der der Erfindung zugrunde liegenden Nukleinsäurenbindenden Oliogomeren bewirkt. Die Internalisierung kann auf verschiedenem Wege, z.B. durch Endocytose oder aktive Transportmechanismen erfolgen.

Die Struktur der Zelloberfläche kann ein Protein, Polypeptid, Kohlenhydrat, Lipid oder eine Kombination daraus sein. Typischerweise wird die Zellaufnahme durch Oberflächenrezeptoren bewirkt. Deshalb kann das Bindungs- und Erkennungs-Agens ein natürlicher oder synthetischer Ligand eines Rezeptors sein.

Der Ligand kann ein Protein, Polypeptid, Kohlenhydrat, Lipid, Steroid oder eine Kombination von diesen sein, ausgestattet mit funktionellen Gruppen, die so angeordnet sind, daß sie durch die Zelloberflächen-Struktur erkannt werden können. Es kann sich auch um eine Komponente oder die Gesamtheit eines biologischen Organismus handeln, z.B. eines Virus, einer Zelle oder um artifizielle Transportsysteme, wie z.B. Liposomen. Es kann sich weiterhin um einen Antikörper oder ein Analogon eines Antikörpers handeln.

Für die Adressierung an unterschiedlichen Zellen müssen verschiedene Liganden eingesetzt werden.

Als Liganden für die Adressierung an Makrophagen kommen bevorzugt Kohlenhydrate, wie z.B. Mannose, Polykationen, wie z.B. Polylysine, Polyarginine, Polyornithine, basische Proteine, wie z.B. Avidin, sowie Glycopeptide, Steroide, Peptide oder Lipopeptide in Frage (G.Y. Chu et al., WO 9304701).

Mit löslichkeitsvermittelnden Gruppen sind funktionelle Gruppen gemeint, die die Löslichkeit in Wasser vermitteln. Dabei kann es sich z.B. um Ester oder Amide von Aminosäuren, Hydroxycarbonsäuren, Aminosulfonsäuren, Hydroxysulfonsäuren oder Diaminen handeln. Bevorzugt sind Amide von Diamincarbonsäuren wie Ornithin, Lysin oder 2,4-Diaminobuttersäure.

In der vorliegenden Anmeldung werden nukleinsäurebindende Oligomere beschrieben, bei welchen die große Variabilität der Bausteine aus DE 4 331 012.5 mit den Eigenschaften der Aminoethylglycin-Bausteine (WO 92/20703 und WO 93/12129, käuflich bei der Firma Millipore) kombiniert wurden. Die zur Oligomerisierung eingesetzten Bausteine wurden in WO 92/20703 und WO 93/12129 beschrieben. Derivate der beschrieben Bausteine sind durch literaturbekannte Reaktionsschritte herstellbar.

### Beschreibung der Experimente

Untersuchungen zu den Hybridisierungseigenschaften, sowie der Nuklease-und Proteasestabilität wurden analog zu den Experimenten in DE 4 331 012.5 durchgeführt. Als zusätzliche Untersuchungsmethode zur Überprüfung der Hybridisierungseigenschaften wurden Kapillarelektrophoresemessungen durchgeführt.

### Allgemeiner Teil:

### Oligomerisierung

Die Verknüpfung der Bausteine zu Oligomeren kann in Lösung erfolgen, wird jedoch vorzugsweise mittels Festphasensynthese durchgeführt (siehe: Merrifield, R.B., J. Am. Chem. Soc., 85, (1963, 2149). Hierzu wird bevorzugt ein Peptidsynthesizer, insbesondere das Modell 431-A der Firma Applied Biosystems, eingesetzt. Als polymere Träger stehen verschiedene kommerziell erhältliche Harze zur Verfügung; bevorzugt werden die PAM-, MBHA- und HMP-Resins der Firma Applied Biosystems verwendet. Die Bausteine werden in Analogie zur konventionellen Peptidsynthese durch gezielte Verwendung einer Schutzgruppenstrategie am N-Terminus, bevorzugt unter Nutzung des Fmoc- oder Boc-Verfahrens, verknüpft. Die Aktivierung erfolgt in der Regel in N-Methyl-2-Pyrrolidon (NMP) durch Umsetzung mit Hydroxybenzotriazol/Dicyclohexylcarbodiimid, oder aber auch unter Nutzung anderer bekannter Aktivierungsverfahren aus der Peptidchemie (beispielsweise Uroniumsalze, wie TBTU, HBTU, BOP, PYBOP usw. in NMP oder anderen Lösungsmitteln, wie DMF, DMSO oder DCM) Die festphasengebundenen Verbindungen werden im Anschluß an die Oligomerisierung durch spezielle Abspaltreagenzien wie HF oder Trifluormethansulfonsäure (Boc-Methode; PAM- oder MBHA-Resin) oder durch Trifluoressigsäure (Fmoc-Methode; HMP-Resin) abgetrennt und durch Filtration vom polymeren Träger entfernt. Bekannte Übersichtsartikel mit detaillierten Beschreibungen des verwendeten Verfahrens sind beispielsweise a) Barany, G., Kneib-Cordonier, N., Mullen, D.G., Int. J. Pept. Protein Res. 30 1987, 705ff und b) Fields, G.B. Noble, R.C., Int. J. Pept. Protein Res., 35, 1990, 161-214. Die Reaktionsprodukte werden durch präparative HPLC, insbesondere unter Nutzung der "reversed phase" Methode bei Verwendung von RP 8 Säulen mit einem Lösungsmittelgemisch, wie beispielsweise einem ansteigenden Gradienten von Trifluoressigsäure in Acetonitril oder Acetonitril/Wasser isoliert. Die Charakterisierung der Verbindungen erfolgt insbesondere durch die Massenspektroskopie.

### Beispiel 1

### Festphasensynthese von NH₂-T₁-T₂-T₁-T₂-T₁-T₂-T₁-T₂-Lys-NH₂

- T₁ =: Aminoethylglycin-Thymin-Baustein, gemaß WO 92/20703
- T₂ =: L-trans-4-Amino-N-[(thymin-1-yl)-acetyl]-prolin-Baustein, gemaß DE 43 31 012.5
Die Oligomerisierung erfolgt unter Nutzung des im Peptidsynthesizer ABI 431-A vorhandenen Programmes für Boc-Small Scale-Reaktionen.

32,5 mg (0,025 mol) MBHA-Resin werden im Reaktionsgefäß vorgelegt. Der Träger wird mit Diisopropylethylamin neutralisiert und mit DCM gewaschen. Die Aktivierung von 1 mmol Boc-Lys(2-chloro-Z)-OH (0,41 g) und jeweils 50 mg T₂-Baustein bzw. 48 mg T₁-Baustein erfolgt durch Umsetzung mit 135 mg (1,0 mmol) Hydroxybenzotriazol und 206 mg (1,0 mmol) Dicyclohexylcarbodiimid in N-Methyl-2-pyrrolidon (NMP). Das Hydroxybenzotriazol wird dabei zur besseren Löslichkeit der Bausteine zusammen mit NMP in der die Aminosäure enthaltenden Kartusche vorgelegt. Die tert.-Butyloxycarbonyl-Schutzgruppe des trägergebundenen Zwischenproduktes wird vor jedem Kupplungsschritt durch Behandlung mit Trifluoressigsäure abgespalten und der Träger anschließend mit Diisopropylethylamin neutralisiert und mit DCM gewaschen. Im Anschluß erfolgt die schrittweise Ankopplung an den polymeren Träger. Nach der letzten Kopplung wird die Boc-Schutzgruppe durch Behandlung mit Trifluoressigsäure entfernt. Wiegen des getrockneten Trägers ergibt 56,1 mg Gewichtszunahme. Die Abspaltung des Oligomeren vom Träger erfolgt durch 2-stündige Behandlung des Polymeren mit 4,5 ml HF und 0,5 ml Anisol bei 0°C im Teflonkolben. Nach der HF-Abspaltung wird der Rückstand 4 mal mit 15 ml absolutem Diethylether gerührt, um anhaftendes Anisol herauszulösen. Nach jeweils 15 Minuten wird der Ether vorsichtig abdekantiert. Nun wird das Oligomer mit 60 ml 30 %iger Essigsäure (4 x 15 ml, je 15 Minuten lang) extrahiert, die Lösung vom Polymer über eine D3-Fritte abgetrennt und das Filtrat lyophilisiert.

Man erhält 46,5 mg Rohprodukt, welches zur Reindarstellung mittels RP-HPLC aufgearbeitet wird. Als stationäres Trennmedium verwendet man eine "Eurosil Bioselect 300 A (5 µm)" Säule unter Nutzung des nachfolgend beschriebenen Elutionssystems:
Eluent A: 0,1 % TFA in Wasser,
Eluent B: 0,1 % TFA in Wasser/Acetonitril (3/7)
Der Gradient wird wie folgt eingestellt:

| Gradient (Min) | Eluent A (%) | Eluent B (%) |
|---|---|---|
| 0,00 | 95 | 05 |
| 30,00 | 40 | 60 |
| 40,00 | 20 | 80 |
| 45,00 | 20 | 80 |
| 50,00 | 95 | 05 |

Die Detektion erfolgt bei 260 nm mittels eines UV-Detektors. Die Oligomere aus Beispiel 2 bis 7 wurden analog Beispiel 1 gereinigt. Die Retentionszeit der Zielsubstanz beträgt 15,2 Minuten.

Das aufgereingte Oligomer wird nach der HPLC lyophilisiert. Man erhält eine Ausbeute an hochreinem Produkt von 35,7 mg (0,0153 mmol, 61,2 % bezogen auf die theor. mögliche Menge; 63,6 % bezogen auf die tatsächliche harzgebundene Menge). Die Charakterisierung des Oligomers erfolgt über Massenspektroskopie (LDI-Methode). Das theroretische Molekulargewicht beträgt 2323 g/mol gemessen werden 2324,0 g/mol.

### Beispiel 2

### Festphasensynthese von NH₂-T₁-T₃-T₁-T₃-T₁-T₃-T₁-T₃-Lys-NH₂

- T₁ =: Aminoethylglycin-Thymin-Baustein, gemäß WO 92/20703
- T₃ =: L-cis-4-Amino-N-[(thymin-1-yl)-acetyl]prolin-Baustein, gemaß DE 43 31 012.5
Die Oligomerisierung erfolgte analog Beispiel 1.

Man erhält 70,0 mg Rohprodukt, welches zur Reindarstellung mittels RP-HPLC aufgearbeitet wird.

Die Retentionszeit der Zielsubstanz beträgt 16,3 Minuten.

Das aufgereingte Oligomer wird nach der HPLC lyophilisiert. Man erhält eine Ausbeute an hochreinem Produkt von 20,4 mg (0,0087 mmol, 34,9 % bezogen auf die theor. mögliche Menge; 36,8 % bezogen auf die tatsächliche harzgebundene Menge). Die Charakterisierung des Oligomers erfolgt über Massenspektroskopie (LDI-Methode). Das theroretische Molekulargewicht beträgt 2323,3 g/mol, gemessen werden 2325,6 g/mol.

### Beispiel 3

### Festphasensynthese von NH₂-(T₁-T₂-T₂-T₂)₃-Lys-NH₂

- T₁ =: Aminoethylglycin-Thymin-Baustein, gemäß WO 92/20703
- T₂ =: L-trans-4-Amino-N-[(thymin-1-yl)-acetyl]prolin, gemäß DE 43 31 012.5
Die Oligomerisierung erfolgte analog Beispiel 1.

Man erhält 65,2 mg Rohprodukt, welches zur Reindarstellung mittels RP-HPLC aufgearbeitet wird.

Die Retentionszeit der Zielsubstanz beträgt 20,5 Minuten. Das aufgereinigte Oligomer wird nach der HPLC lyophilisiert. Man erhält eine Ausbeute an hochreinem Produkt von 15,9 mg (0,0046 mmol; 18,4 % bezogen auf die theor. mögliche Menge; 21,0 % bezogen auf die tatsächlich harzgebundene Menge). Die Charakterisierung des Oligomers erfolgt über Massenspektroskopie (LDI-Methode, ohne Zusatz eines Massenstandards gemessen). Das theoretische Molekulargewicht beträgt 3448,3 g/mol, gemessen werden 3449 g/mol.

### Beispiel 4

### Festphasensynthese von NH₂-(T₁-T₂-T₂)₄-Lys-NH₂

- T₁ =: Aminoethylglycin-Thymin-Baustein, gemäß WO 92/20703
- T₂ =: L-trans-4-Amino-N-[(thymin-1-yl)-acetyl]prolin, gemäß DE 43 31 012.5
Die Oligomerisierung erfolgte analog Beispiel 1.

Man erhält 60,2 mg Rohprodukt, welches zur Reindarstellung mittels RP-HPLC aufgearbeitet wird.

Die Retentionszeit der Zielsubstanz beträgt 19,6 Minuten. Das aufgereinigte Oligomer wird nach der HPLC lyophilisiert. Man erhält eine Ausbeute an hochreinem Produkt von 11,8 mg (0,0034 mmol; 13,7 % bezogen auf die theor. mögliche Menge; 15,7 % bezogen auf die tatsächlich harzgebundene Menge). Die Charakterisierung des Oligomers erfolgt über Massenspektroskopie (LDI-Methode, kein Zusatz eines internen Massenstandards). Das theoretische Molekulargewicht beträgt 3436,3 g/mol, gemessen werden 3442 g/mol.

### Beispiel 5

### Festphasensynthese von NH₂-(T₂-T₁)₄-Lys-NH₂

- T₁ =: Aminoethylglycin-Thymin-Baustein, gemaß WO 92/20703
- T₂ =: L-trans-4-Amino-N-[(thymin-1-yl)-acetyl]prolin, gemäß DE 43 31 012.5
Die Oligomerisierung erfolgte analog Beispiel 1.

Man erhält 59,3 mg Rohprodukt, welches zur Reindarstellung mittels RP-HPLC aufgearbeitet wird.

Die Retentionszeit der Zielsubstanz beträgt 17,4 Minuten. Das aufgereinigte Oligomer wird nach der HPLC lyophilisiert. Man erhält eine Ausbeute an hochreinem Produkt von 44,5 mg (0,0192 mmol; 76,6 % bezogen auf die theor. mögliche Menge; 66,0 % bezogen auf die tatsächlich harzgebundene Menge). Die Charakterisierung des Oligomers erfolgt über Massenspektroskopie (LDI-Methode, kein Zusatz eines internen Massenstandards). Das theoretische Molekulargewicht beträgt 2323,2 g/mol, gemessen werden 2325 g/mol.

### Beispiel 6

### Festphasensynthese von NH₂-(T₁-T₄)₆-Lys-NH₂

- T₁ =: Aminoethylglycin-Thymin-Baustein, gemäß WO 92/20703
- T₄ =: D-trans-4-Amino-N-[(thymin-1-yl)-acetyl]prolin, gemäß DE 43 31 012.5
Die Oligomerisierung erfolgte analog Beispiel 1.

Man erhält 89 mg Rohprodukt, welches zur Reindarstellung mittels RP-HPLC aufgearbeitet wird.

Die Retentionszeit der Zielsubstanz beträgt 23,0 Minuten. Das aufgereinigte Oligomer wird nach der HPLC lyophilisiert. Man erhält eine Ausbeute an hochreinem Produkt von 78 mg (0,022 mmol; 91 % bezogen auf die theor. mögliche Menge; 90 % bezogen auf die tatsächlich harzgebundene Menge). Die Charakterisierung des Oligomers erfolgt über Massenspektroskopie (LDI-Methode, kein Zusatz eines internen Massenstandards). Das theoretische Molekulargewicht beträgt 3412,3 g/mol, gemessen werden 3417 g/mol.

### Beispiel 7

### Festphasensynthese von NH₂-(T₁-T₄-T₄)₄-Lys-NH₂

- T₁ =: Aminoethylglycin-Thymin-Baustein, gemäß WO 92/20703
- T₄ =: D-trans-4-Amino-N-[(thymin-1-yl)-acetyl]prolin, gemäß DE 43 31 012.5
Die Oligomerisierung erfolgte analog Beispiel 1.

Man erhält 81,0 mg Rohprodukt, welches zur Reindarstellung mittels RP-HPLC aufgearbeitet wird.

Die Retentionszeit der Zielsubstanz beträgt 22,0 Minuten. Das aufgereinigte Oligomer wird nach der HPLC lyophilisiert. Man erhält eine Ausbeute an hochreinem Produkt von 70,7 mg (0,02 mmol; 81,5 % bezogen auf die theor. mögliche Menge; 63,1 % bezogen auf die tatsächlich harzgebundene Menge). Die Charakterisierung des Oligomers erfolgt über Massenspektroskopie (LDI-Methode, kein Zusatz eines internen Massenstandards). Das theoretische Molekulargewicht beträgt 3436,3 g/mol, gemessen werden 3462 g/mol.

### Beispiel 8

### Festphasensynthese von NH₂-(T₁-T₄-T₂)₄-Lys-NH₂

- T₁ =: Aminoethylglycin-Thymin-Baustein, gemäß WO 92/20703
- T₂ =: L-trans-4-Amino-N-[(thymin-1-yl)-acetyl]prolin, gemäß DE 43 31 012.5
- T₄ =: D-trans-4-Amino-N-[(thymin-1-yl)-acetyl]prolin, gemäß DE 43 31 012.5
Die Oligomerisierung erfolgte analog Beispiel 1.

Man erhält 59,0 mg Rohprodukt, welches zur Reindarstellung mittels RP-HPLC aufgearbeitet wird.

Die Retentionszeit der Zielsubstanz beträgt 20,0 Minuten. Das aufgereinigte Oligomer wird nach der HPLC lyophilisiert. Man erhält eine Ausbeute an hochreinem Produkt von 52,2 mg (0,015 mmol; 60,8 % bezogen auf die theor. mögliche Menge; 61,3 % bezogen auf die tatsächlich harzgebundene Menge). Die Charakterisierung des Oligomers erfolgt über Massenspektroskopie (LDI-Methode). Das theoretische Molekulargewicht beträgt 3436,3 g/mol, gemessen werden 3440 g/mol.

### Test auf biologische Stabilität gegenüber Proteasen und Nucleasen

### Beispiel 9

### Stabilität gegenüber Proteinase K:

Zu jeweils 75 µg der Verbindungen aus Beispiel 1 und Beispiel 2 in je 75 µl bidest. Wasser wurden jeweils 20 µl 1M Tris/HCl (pH 7,5), 80 µl 50 mM Calciumchlorid-Lösung und 1 U Proteinase K (Serva) hinzugefügt und die Mischung 3 Stunden bei 37°C inkubiert. Anschließend erfolgte eine HPLC-Untersuchung (reversed phase, Eurosil-Bioselect, Eluent: 5-70 % 0,1 % Trifluoressigsäure in Wasser/Acetonitril (3/7) gegen 0,1 % Trifluoressigsäure in Wasser) des jeweiligen Reaktionsgemisches. In keinem Fall war ein neuentstandenes Abbauprodukt detektierbar, wohingegen das Signal der Verbindungen aus Beispiel 1 und Beispiel 2 weiterhin vorhanden war. Die Verbindungen sind damit gegenüber Proteinase K stabil.

### Beispiel 10

### Stabilität gegenüber S1-Nuclease:

Zu jeweils 75 µg der Verbindungen aus Beispiel 1 und Beispiel 2 in je 75 µl bidest. Wasser wurden jeweils 20 µl Nucleasepuffer (Promega) und 4 µl S1-Nuclease (Promega, 50 U/ml) und die Mischung 3 Stunden bei 37°C inkubiert. Anschließend erfolgte eine HPLC Untersuchung (reversed phase, Eurosil-Bioselect, Eluent: 5-70 % 0,1 % Trifluoressigsäure in Wasser/Acetonitril (3/7) gegen 0,1 % Trifluoressigsäure in Wasser) des jeweiligen Reaktionsgemisches. In keinem Fall war ein neuentstandenes Abbauprodukt detektierbar, wohingegen das Signal der Verbindungen aus Beispiel 1 und Beispiel 2 weiterhin vorhanden war. Die Verbindungen sind damit gegenüber S1-Nuclease stabil.

### Beispiel 11

Bestimmung der Annealing Temperatur ausgewählter Oligomerer mit A₈- bzw. A₁₂-DNA-Strang.

Die entsprechenden DNA-Stränge wurden auf einem Applied Biosystems "ABI 380B" DNA-Synthesizer mit Hilfe der Phosphoramiditmethode nach dem small scale Cycle des Herstellers Applied Biosystems hergestellt.

Die Bestimmung der Annealing Temperatur erfolgte unter Nutzung eines Perkin Elmer "Lambda Bio" UV-Vis-Spektrometers unter Verwendung der vom Hersteller angegebenen Methode "PE-TEMP".

Hierzu wird soviel PNA in 700 µl Wasser gelöst, bis sich eine Absorbtion von 0,3 ergibt. Desgleichen erfolgt mit dem korrespondierenden DNA-Strang. Anschließend werden beide Stränge vereint und das Wasservolumen auf 1,5 ml erhöht. Die vereinigten Stränge werden nun 5 Minuten lang bei 95°C erhitzt und dann über Nacht in einem Styroporgefäß langsam abgekühlt.

Anschließend werden die Doppelstränge gemaß der Methode "PE-Temp" im Temperaturbereich von 20°C bis 80°C auf ihre Absorbtion hin untersucht. Der Umkehrpunkt (Maximum der 1. Ableitung) der entstehenden Absorptionskurve entspricht dann der gemessenen Annealing-Temperatur auf der Temperaturskala.

Vermessen wurden folgende Oligomere:

| Oligomere aus Beispiel | DNA-Strang | Annealing Temp. in °C |
|---|---|---|
| 1 | A₈ | 47,0 |
| 2 | A₈ | 27,0 |
| 3 | A₁₂ | 34,4 |
| 4 | A₁₂ | 48,8 |
| 5 | A₈ | 46,0 |
| 6 | A₁₂ | 27,0 |

### Beispielversuch 12

### Nachweis der Strangverdrängung in doppelsträngiger Plasmid-DNA durch nukleinsäurebindende Oligomere

Nachfolgend wird der Test zum experimentiellen Nachweis der DNA Doppelstrangverdrängung durch die nukleinsäurebindenden Oligomere beschrieben. Diese Eigenschaft der DNA Doppelstrangverdrängung ist mit Ribosephosphat-, Ribosemethylphosphonat-, Ribosephosphorothioat-Backbone und anderen nukleinsäureähnlichen Backbonetypen nicht zu erreichen.

Die im Beispiel eingesetzte Plasmid-DNA ist ein Modell-Substrat für den Nachweis der DNA Doppelsträngverdrängung. Andere Plasmide, die entsprechende Zielsequenzen mit komplementärer Basensequenz zu den zu prüfenden nukleinsäurebindenden Oligomere enthalten, sind in gleicher Weise für den Test verwendbar.

Doppelsträngige, zirkuläre Plasmid-DNA von 4880 Basenpaaren Länge, die zwei Poly-Adenin Sequenzbereiche mit mindestens neun aufeinanderfolgenden Adeninnukleotiden im Abstand von 1150 Basenpaaren enthält, wird in den hier beschriebenen Tests verwendet.

Sieben parallel angesetzte Proben, bezeichnet (1-7), enthielten je 1,0 µg ungeschnittene Plasmid-DNA in 14 µl H₂O. Den Proben 3 bis 7 wurden je 1 µl Lösung von 0,0001 µg, 0,001µg, 0,01 µg, 0,1 µg und 1,0 µg nukleinsäurebindendes Oligomer aus den Beispielen 1 bis 5 zugesetzt und in geschlossenen Eppendorfreaktionsgefäßen 45 min bei 37°C inkubiert. Anschließend wurde in alle Proben 4 µl Puffer (250 mM Na-Acetat, 1M NaCl, 2,5 % Glycerin, 5 mM ZnCl₂, pH 4,4) und in die Proben 2 bis 7 zusätzlich je 1 µl S1-Nuclease aus Aspergillus oryzae, (Fa. Boehringer Mannheim) mit einer Aktivität von 10 U/µl gegeben. Nach einer Inkubation von 15 Minuten bei 30°C wurden die Proben auf Eis gesetzt, 1 µl 0,5 M EDTA und 3 µl Auftragspuffer (50 % Glycerin, 0,25 % Bromphenolblau in 40 mM Tris-HCl, 20 mM Natriumacetat, 1 mM EDTA, pH = 7,2) zugegeben und ohne Zeitverzug die Proben über 1,2 %-Agarosegele elektrophoretisch aufgetrennt und nach Anfärbung mit Ethidiumbromid die Größe der entstandenden Plasmid-Fragmente im Gel im Vergleich zu einem Molekulargewichtsstandard (1 kb Leiter, Fa. Gibco-BRL, D-7514 Eggenstein) auf dem Transilluminator bei 254 nm UV-Licht bestimmt.

Es zeigte sich, daß in den Proben mit einer Konzentration > 0,001 µg/Test (≙ 4,3.10⁻⁷ M) der Oligomere aus Beispiel 1 (Proben 5-7) DNA-Fragmente von 4880 Basenpaaren (Plasmid-Lineaerisierung), und 3270, 2570 sowie 1150 Basenpaaren durch die S1-Nuklease-Reaktion erzeugt wurden. Durch diese Fragemente wird die sequenzselektive Bindung des Oktamers aus Beispiel 1 an doppelsträngige DNA zur Strangverdrängung und anschließende S-1 Nukleasespaltung nachgewiesen.

Mit einem modifizierten Testansatz bei dem in die Proben anstelle der zirkulären, ungeschnittenen Plasmid-DNA eine Plasmid-DNA gegeben wurde, die durch Restriktionsendonuklaeseverdau in unmittelbarer Nachbarschaft von einer der beiden Poly-Adenin Sequenzbereiche linearisiert war, waren ebenfalls in Proben 5-7 DNA-Fragmente von 3730, 4480, 2570 und 1150 Basenpaaren Länge nachweisbar, die die sequenzselektive Doppelstrangverdrängung belegen.

Weiterhin wurde auch bei höherer Salzkonzentration mit 5 mM Tris HCl, 1 mM Mg Cl₂, 10 mM NaCl, pH 7,0 anstelle von Wasser DNA Doppelstrangverdrängung mit dem Oktamer aus Beispiel 1 an doppelsträngiger Plasmid DNA nachgewiesen.

Beispielhaft wurde weiterhin mit den Oligomeren aus den Beispielen 3 bis 5 eine vergleichbare DNA-Doppelstrangverdrängung in der hier beschriebenen Testanordnung nachgewiesen. Auch die Oligomere der Beispiele 3 bis 5 führten bei 0,01 µg bis 0,001 µg im Test (ca. 5 · 10⁻⁶ M bis 5 · 10⁻⁷ M) zu einer sequenzselektiven Spaltung doppelsträngiger DNA durch S1-Nuclease aus Aspergillus oryzae.

Mit diesen Testreihen war die konzentrationsabhängige und sequenzselektive Bindung des Oligomers aus den Beispielen 1 bis 5 an doppelsträngige DNA und der Nachweis der dadurch entstehenden Einzelstrang-DNA durch S1-Nukleaseverdau (bei hohen Salzkonzentrationen zur einzelstrangspezifischen Aktivität von S1-Nuklease) nachweisbar.

### Beispiel 13

### Gel-shift-Analysen

Gelshift-Analysen können das Hybridisierungspotential von Nukleinsäurebindenden Oligomeren gegenüber normalen Diesteroligomeren qualitativ und quantitativ belegen. Hierzu wird einzelsträngige DNA von entsprechender Basensequenz mit dem zu untersuchenden Oligomer inkubiert und anschließend in einer Gelelektrophorese aufgetrennt. Hybridisierte DNA zeigt gegenüber freier DNA einen deutlichen Shift im Gel. Durch Variation der Konzentration der Nukleinsäure-bindenden Oligomere können quantitative Aussagen über das Maß der Hybridisierung gemacht werden.

### Testdurchführung:

1 µg Diesteroligonukleotid von entsprechender Basensequenz wird mit Polynukleotidkinase und γ-ATP am 5'-Ende in gängiger Weise in einem Volumen von 10 µl markiert (Sambrook, Fritsch, Maniatis: Molecular Cloning, A Laboratory Manual, Cold Spring Harbor, 1989). Nach der Markierung wird die Probe zur Denaturierung des Enzyms bei 70°C für 10 Min. erhitzt und anschließend mit 9 µg nicht markiertem Oligomer vermischt. 1 µl dieses Ansatzes wird mit einer gewünschten Menge zu testendem Nukleinsäure-bindenden Oligomer versetzt (1-10 µg) und in einem Volumen von 20 µl 30 Min. bei 22°C (Raumtemperatur) inkubiert (Hybridisierung). Danach wird die Probe 30 Min. auf Eis gestellt. Ein nicht hybridisiertes markiertes Oligomer wird in gleicher Weise behandelt und dient als Kontrolle. Die Proben werden auf ein 15 % Polyacrylamidgel mit 1 x Tris-Borat-EDTA-Puffer aufgetragen. Das Gel und der Puffer werden im Kühlschrank (8°C) vorgekühlt, die Elektrophorese wird über Nacht mit 55 V im Kühlschrank laufen gelassen. Nach der Elektrophorese wird ein Autoradiogramm auf AGFA-Film erstellt (Exponierzeiten 1 bis 16 Stunden).

### Ergebnisse:

Die Verbindungen aus den Beispielen 1, 3, 4 und 6 zeigen schon in äquimolarer Konzentration zu einem Diester deutliche Gelshifts, die schon in 5 bis 10 x Überschuß vollständig sind und damit die sehr guten Hybridisierungseigenschaften dieser Verbindungen belegen. Die Verbindungen aus den Beispielen 5 und 7 zeigen demgegenüber ein leicht geringeres Hybridisierungspotential.

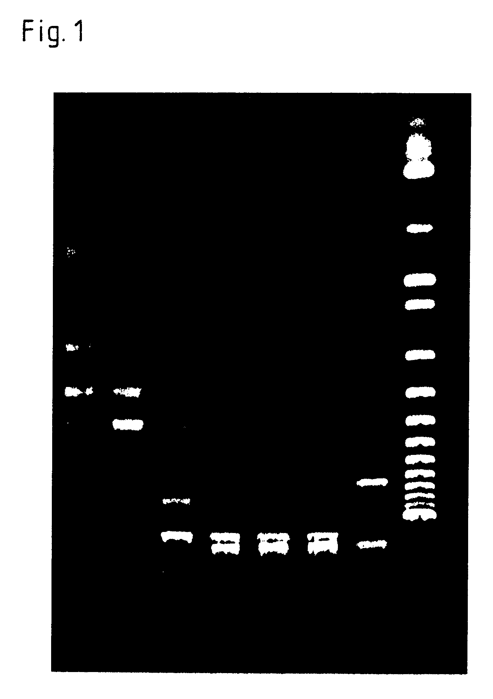

### Legende zu Abbildung 1:

Agarosegelelektrophorese der Testansätze aus Beispiel 5 zum Nachweis der DNA Doppelstrangverdrängung nach Inkubation der Plasmid DNA mit dem DNA bindenden Oktamer aus Beispiel 1 und anschließender Reaktion mit S1 Nuklease. Das mit Ethidiumbromid gefärbte Gel zeigt in den Spuren:
- 0 =: Molekulargewichtsstandard für DNA Doppelstrangfragmente
- 1 =: ungeschnittene Plasmid-DNA (Substrat für jeden Testansatz identisch)
- 2 =: wie 1, jedoch mit 10 U Nuklease S1
- 3-7 =: wie 2, jedoch zusätzlich mit 0,0001; 0,001, 0,01; 0,1 und 1,0 µg DNA bindendes Oktamer aus Beispiel 1 im Test
Bei einer Konzentration > 4,3 x 10⁻⁷ M des Oktamers aus Beispiel 1 ist die DNA Doppelstrandverdrängung im Test nachweisbar.

### Beispiel 14 Kapillarelektrophorese

### Einführung:

Die Überführung der Hybridisierungseigenschaften von Oligonukleotidanaloga erfolgt häufig über den Schmelzpunkt T_{M} der komplementären Oligonukleotide. In jüngster Vergangenheit wurde auch die Kapillargelelektrophorese (CE mit festem Gel in der Kapillare) zur Bestimmung der PNA-DNA-Bindung eingesetzt (Rose, D.J. Anal. Chem. (1993), 65, 3545-3549). für die Trennung von DNA-Fragmenten konnten auch dynamische Flüssiggele erfolgreich eingesetzt werden. (Barron, A.E.; Soane, D.S. & Blanch, H.W. J. Chromatogr. (1993), 652, 3-16).

Die dynamische Gelkapillarelektrophorese (DGCE) ist durch die hohe Reproduzierbarkeit und Verfügbarkeit für die Trennung oligomerer DNA-Nukleotide besonders geeignet.

### Experimentelles:

### Kapillarelektrophorese (CE)

Die Untersuchungen wurden an einem ABI 270A-HT (Applied-Biosystems, Weiterstadt), es ist auch jedes andere CE-Gerät mit UV-Detektor geeignet, durchgeführt. Die Bedingungen stehen in Tabelle 1. Die Daten wurden über einen AD-Wandler auf einen PC übertragen und mit der HPCHEM-Software (Hewlett-Packard, Waldbronn) aufgezeichnet und ausgewertet.

### Hybridisierung:

Das nukleinsäurebindende Oktamer aus Beispiel 1 wurde in einer konstanten Konzentration von 45 µM in TRIS x HCl (5 mM, 0,1 ml) mit dem komplementären DNA-Oktameren δ(A)₈ in unterschiedlichen Verhältnissen hybridisiert. Gewählt wurden die Verhältnisse 0,2/1, 0,25/1, 0,33/1, 0,5/1, 1/1, 2/1 und 3/1.

Die Proben wurden parallel und nach standardisiertem Verfahren bei 93°C für 5 min erhitzt und dann allmählich auf RT abgekühlt und nach einer 1:5 Verdünnung mit Wasser direkt gemessen.

**Tabelle 1**

| **Meßparameter für die Hybridisierungsbestimmung mit DGCE** | | |
|---|---|---|
| Puffer : | 100 nM Tris/Borat + 0,5% Dextran pH 8,5 | |
| Kapillare : | Fused Silica (ABI) | |
| | Längen: Gesamt: | 50 cm Effektiv: 29 cm |
| | Innendurchmesser: | 50 µm |
| Messparameter: | Vakuuminjektion: | 1,7 · 10⁴ Pa |
| | Injektionszeit: | 3 sec |
| | Spannung: | 25 kV |
| | Temperatur: | 30°C |
| | Laufzeit: | 10 min |
| Detektion: | | 257 nm |
| Säulenkonditionierung: | Spülvakuum: | 6,8 · 10⁴ Pa |
| | 1. Spülschritt: | 2 min 0,1 N NaOH |
| | 2. Spülschritt: | 6 min Puffer |

### Ergebnisse und Diskussion:

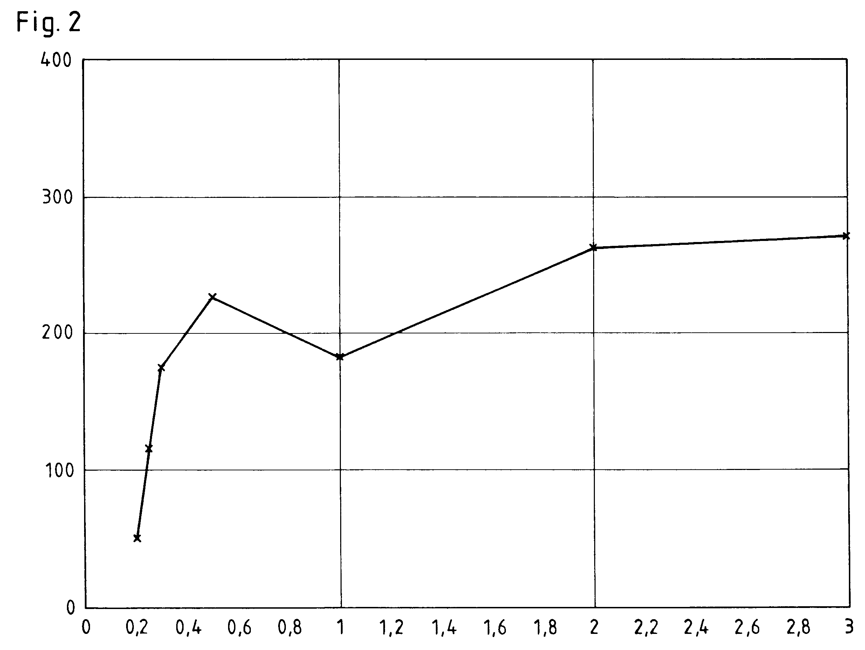

Die Hybridisierungsausbeute in Abhängikeit von der Konzentration an komplementärem DNA-Oktamer δ(A)₈ zeigt einen starken Anstieg der Hybridisierungsausbeute bis zu einem Verhältnis von 1:0,5. Danach bleibt das Hybridisierungsprodukt trotz zunehmender Konzentration an komplementären Oktamer δ(A)₈ relativ konstant (Abb. 2). Das Verhältnis von 1:0,5 weist auf eine 2:1 Hybridisierung des nukleinsäurebindenden Oktamers aus Beispiel 1 mit dem komplementären Oktamer δ(A)₈ hin.

### Legende zu Abbildung 2:

In der Abbildung wird die maximale Peakhöhe des Hybridisierungsprodukts des nukleinsäurebindenden Oktamers aus Beispiel 1 mit dem komplementären Oktamer δ(A)₈ ε (y-Achse) gegen die relative Konzentration an komplementärem Oktamer δ(A)₈ (x-Achse) aufgetragen. Die maximale Hybridisierung wird bereits bei einem Verhältnis von 2:1 an nukleinsäurebindendes Oktamer aus Beispiel 1 zu komplementrärem Oktamer δ(A)₈ erreicht.

## Patentansprüche

1. Verbindungen der allgemeinen Formel (I), 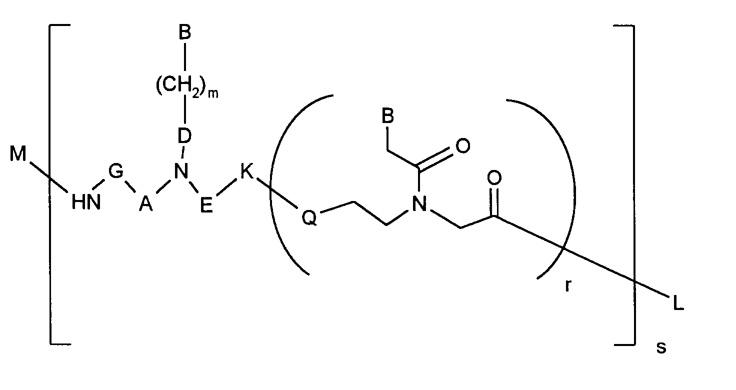 in der
A für -(CH₂)ₙ- oder -CO- steht,
B für alle natürlichen oder unnatürlichen Nukleobasen, wie z.B. Thymin, Uracil, Cytosin, Adenin, Guanin, Hypoxanthin, oder durch chemische Modifikation von diesen abgeleitete Derivate oder halogenierte Vorstufen von diesen, gegebenenfalls an den Aminogruppen mit Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert oder mit freien Aminogruppen steht,
D für -(CO)ₚ- steht,
E und G unabhängig voneinander für -CHR- stehen, wobei
R Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Tyrosin, Histidin, Tryptophan, Lysin, Ornithin, Asparagin, Asparaginsäure, Glutamin, Glutaminsäure, Arginin, Prolin, Hydroxyprolin, Sarcosin, Sarcosin, Dehydroaminosäuren, wie z.B. Dehydroalanin, Dehydro-α-aminobuttersäure, anderen unnatürlichen Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Iodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, in ihrer D- oder L-Form oder gegebenenenfalls
E und G über eine Kette -(CHR')_{q}- miteinander verknüpft sind,
K für -CO-, -SO₂- oder -CH₂- steht,
L ein Carrier-System, Reporter-Ligand, eine löslichkeitsvermittelnde Gruppe oder Wasserstoff sein kann,
M unabhängig von L ein Carrier-System, Reporter-Ligand, eine löslichkeitsvermittelnde Gruppe oder Wasserstoff sein kann,
Q für NH, O, S, NR'' steht,
R' unabhängig voneinander aus einer Gruppe bestehend aus H, OH, SH, NH₂, NHR'', N₃, Alkyl (wobei Alkyl = Methyl, Ethyl, n-Propyl, n-Butyl, iso-Butyl, tert.-Butyl oder längerkettigen verzweigten oder unverzweigten, gesättigten oder ungesättigten Alkylketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) ausgewählt werden kann,
R'' für Schutzgruppen wie z.B. Boc, Fmoc, Z, Pyoc, Alloc oder andere in der Peptidchemie üblichen Schutzgruppen oder aber für Alkylsubstitution (wobei Alkyl = Methyl, Ethyl, n-Propyl, n-Butyl, iso-Butyl, tert.-Butyl oder längerkettigen verzweigten oder unverzweigten, gesättigten oder ungesättigten Alyklketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) steht,
m 0, 1, 2 oder 3 sein kann,
n 0, 1, 2, 3 oder 4 sein kann,
p 0, 1 oder 2 sein kann,
q 0, 1 oder 2 sein kann, und
r 0, oder 1 sein kann, und
s Werte zwischen 1 und 30 annehmen kann.

2. Verbindungen der allgemeinen Formel (I) gemäß Anspruch 1,
in der
A für -(CH₂)ₙ- oder -CO- steht,
B für alle natürlichen Nukleobasen, wie z.B. Thymin, Uracil, Cytosin, Adenin, Guanin oder Hypoxanthin oder halogenierte Vorstufen von diesen, gegebenenfalls an den Aminogruppen substituiert durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen oder mit freier Aminogruppe steht,
D für -(CO)ₚ- steht,
E und G unabhängig voneinander für -CHR- stehen, wobei
R Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Ornithin, Asparagin, Asparaginsäure, Glutamin, Glutaminsäure, Arginin, Prolin, Hydroxypyrolin, Sarcosin, Dehydroaminosäuren, wie z.B. Dehydroalanin, Dehydro-α-aminobuttersäure, anderen unnatürlichen Aminosäuren wie Phenylglycin, 2-Pyridylalanin, 3-Pyridylalanin, 4-Pyridylalanin, 1-Naphthyalanin oder 2-Naphthylalanin, gegebenenfalls mit Schutzgruppen, in ihrer D- oder L-Form oder gegebenenenfalls
E und G über eine Kette -(CHR')_{q} miteinander verknüpft sind,
K -CO-, SO₂- oder -CH₂- sein kann,
L ein Carrier-System, Reporter-Ligand, eine löslichkeitsvermittelnde Gruppe oder Wasserstoff sein kann,
M unabhängig von L ein Carrier-System, Reporter-Ligand, eine löslichkeitsvermittelnde Gruppe oder Wasserstoff sein kann,
Q für NH, O, NR'' steht,
R' unabhängig voneinander aus einer Gruppe bestehend aus H, OH, SH, NH₂, NHR'', N₃, Alkyl (wobei Alkyl = Methyl, Ethyl, n-Propyl, n-Butyl, iso-Butyl, tert.-Butyl oder längerkettigen verzweigten oder unverzweigten, gesättigten oder ungesättigten Alkylketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) ausgewählt werden kann,
R'' für Schutzgruppen wie z.B. Boc, Fmoc, Z, Pyoc, Alloc oder andere in der Peptidchemie üblichen Schutzgruppen oder aber für Alkylsubstitution (wobei Alkyl = Methyl, Ethyl, n-Propyl, n-Butyl, iso-Butyl, tert.-Butyl oder längerkettigen verzweigten oder unverzweigten, gesättigten oder ungesättigten Alyklketten sein kann), Aryl (wobei Aryl = Phenyl, 2-Pyridyl oder 4-Pyridyl sein kann) oder Aralkyl (wobei Aralkyl = Benzyl, Naphthylmethyl oder β-Naphthylmethyl sein kann) steht,
m 0, 1, 2 oder 3 sein kann,
n 0, 1, 2 oder 3 sein kann,
p 0 oder 1 sein kann,
q 0, 1 oder 2 sein kann,
r 0 oder 1 sein kann, und
s Werte zwischen 3 und 20 annehmen kann.

3. Arzneimittel enthaltend eine oder mehrere Verbindungen aus den Ansprüchen 1 und 2.
